# EUROPEAN PATENT APPLICATION

(11) **EP 2 057 905 A1**
(43) Date of publication of application: **13.05.2009**
(21) Application number: 07120500.9
(22) Date of filing: 12.11.2007
(51) Int. Cl.: A23L 1/03, A61K 31/122, A61K 31/198, A61K 31/225, A61K 31/375, A61K 31/455, A61K 31/525, A61P 3/06, A61P 15/00, A61P 15/10, A61P 9/10, A61P 43/00

(54) **Composition for moderating Triglyceride and Cholesterol Levels**

(71) Applicant: TIMA Foundation, 9496 Balzers (LI)
(72) Inventor: Inufusa, Haruhiko, Osaka-City Osaka 542-0081 (JP)
(74) Representative: Dehmel, Albrecht

(57) **Abstract**

The present invention relates to the use of a composition comprising vitamin C, glutamic acid and/or glutamine, cysteine, riboflavin, succinic acid, fumaric acid, coenzyme Q10 and niacin and/or prodrugs of these substances and/or salts of these substances for the manufacture of a food supplement or medicament for prophylaxis and/or treatment of hypertriglyceridemia and hypercholesterolemia and related disease states as well as erectile dysfunction.

## Description

The present invention relates to the use of a composition comprising vitamin C, glutamic acid and/or glutamine, cysteine, riboflavin, succinic acid, fumaric acid, coenzyme Q10 and niacin and/or prodrugs of these substances and/or salts of these substances for the manufacture of a food supplement or medicament for prophylaxis and/or treatment of hypertriglyceridemia and hypercholesterolemia and related disease states as well as erectile dysfunction.

Hypertriglyceridemia is associated with various diseases conditions. Causes of hypertriglyceridemia can be, for example, idiopathic, pancreatitis, obesity, high sugar diet, diabetes mellitus, insulin resistance, alcohol abuse, chronic renal failure, glycogen storage diseases etc. All of these diseases can give rise to elevated triglyceride levels in the blood. In a healthy human subject the levels of blood triglyceride are under strict control and kept (as rough point of reference) below about 180 mg/dl. The latter is only a rough point of reference because this value can vary with ethnic background, age and gender. Due to these variations, the onset of a pathologic condition may be reached in individual cases already with lower values or higher values for blood triglyceride levels. However, such variations are very well within the knowledge of the person skilled in the art and an accurate diagnosis can be established individually for each patient, taking into account all relevant parameters. The consequence of hypertriglyceridemia is for example an increased risk for development of atherosclerosis, obesity and/or pancreatitis. These disease states can again give rise to further complications, e.g. atherosclerosis leads to several kinds of cardiovascular diseases comprising heart diseases, stroke, diabetes mellitus, insulin resistance, metabolic syndrome, high blood pressure and obesity. In all these disease states triglyceride levels can exceed the above mentioned reference value, for the most of them even markedly.

Sources of triglyceride are, e.g., vegetable oil and animal fats. Structurally, triglycerides are composed of glycerol and various fatty acids.

As mentioned above, hypertriglyceridemia is a main cause for obesity. Interestingly, a follow up disease of obesity is diabetes. High triglyceride levels lead to an increase in fat cells in the body. However, the affinity of fat cells to insulin is the highest in comparison to all other cells in the body. For instance, the affinity of fat cells full of triglycerides to insulin is nearly ten-times higher than that of muscle cells. Therefore, in patients suffering from obesity, fat cells bind most of the circulating plasma insulin and consequently other cells suffer relative insulin deficiency. This abnormal increased insulin consumption results in a capacity overload of insulin-secreting pancreatic beta cells, leading to their apoptotic cell death. This in turn leads to a diabetic disease condition.

Hypercholesterolemia is alike hypertriglyceridemia associated with various diseases conditions. Literally, hypercholesterolemia indicates a high blood cholesterol level. It is a metabolic derangement that is frequently secondary to many primary diseases. Causes for hypercholesterolemia can be for example diabetes, nephritic syndrome, overweight, gout, alcohol abuse, hypothyroidism, anorexia nervosa, Zieve's syndrome, pregnancy and metabolic syndrome. There is also a genetic form of this metabolic derangement resulting in familial hypercholesterolemia. In addition, hypercholesterolemia contributes on its own directly to the pathology of many forms of disease conditions, e.g. atherosclerosis, cardiovascular diseases, myocardial infarction, stroke, angina pectoris, ischemic colitis, transient ischemic attacks, and/or peripheral artery disease.

Usually, patients with blood cholesterol levels above about 200 mg/dl are considered to exhibit pathologic hypercholesterolemia. Again, such value is only a rough statistical indicator for hypercholesterolemia and a reliable diagnosis will have to be set up by competent medical staff.

In the blood there are specific carriers for cholesterol transport. Low-density lipoprotein (LDL, atherosclerotic lipoprotein) is a transporter for cholesterol and triglycerides from the liver to the peripheral tissues. In contrast, high-density lipoprotein (HDL, anti-atherosclerotic lipoprotein) is the carrier for cholesterol back from the peripheral tissues to the liver. Epidemiological data show unequivocally that elevated blood or plasma LDL cholesterol concentration is a major risk factor for, e.g. atherosclerosis, which can lead to coronary heart disease and vascular disease in the brain. Currently, LDL levels below 100 mg/dl are considered optimal resulting in the lowest risk for development of heart disease. With increasing values above 100 mg/dl the risk for development of heart disease increases in parallel. Concentrations in blood HDL of < about 40 (men) or < about 50 (women) mg/dl are considered to reflect a heightened risk for development of heart disease. Therefore, therapies aiming at the decrease of LDL and/or the increase in HDL are important for, e.g., atherosclerosis management.

It should also be noted that there is an inverse relation between the level of plasma triglyceride and that of HDL-cholesterol: Very low-density lipoprotein (VLDL) mainly transports triglycerides from the liver to the peripheral tissues. The plasma enzyme, cholesterol ester transfer protein (CETP), transfers cholesterol-ester from HDL to VLDL, and triglyceride from VLDL to HDL. An increase in plasma triglyceride levels causes a decrease in HDL levels.

The prior art has attempted to provide treatment for diseases like obesity, atherosclerosis etc. The initial therapy in all types of hyperlipidemia is an appropriate diet including restriction of caloric intake, fat intake and cholesterol intake. However, very often, it takes time (usually more than 5-6 months) until the participants recognize the loss of body weight and the improvements of their plasma triacylglycerol and cholesterol levels. Even once these levels are normalized, the great majority of participants regain body weight and fall back to the hyperlipidemia when followed for 3-5 years. Exercise may be the most effective prescription for weight loss, if the participants continuously follow the prescription.

There are three categories of drugs for the treatment of hypertriglyceridemia (including obesity). In the first category, fat absorption inhibitors by blocking pancreatic triglyceride lipase in the intestine, such as Orlistat. However, the long-term efficacy and safety of fat absorption inhibitors has yet to be determined. In the second category are thermogenic agents which increase basal metabolism rate and "fat burning", such as thyroid hormones and 3-adrenergic agonists. Currently, there are no approved agents for clinical use. The third category are anorectics, i.e. appetite suppressant drugs (suppression of food intake), such as serotonin agonists, sympathomimetic agents and leptin. In some cases, serotonin agonists and sympathomimetic agents are effective for weight loss and improvement of hypertriglyceridemia. However, they have psychoactive side-effects. Furthermore, for loss of body weight numerous food supplements claiming fat burning effects are available on the market.

With regard to the treatment of hypercholesterolemia (including atherosclerosis and cardiovascular diseases) there are five categories of drugs; HMG-CoA reductase inhibitors (inhibitors for cholesterol biosynthesis; so-called "statins"), cholesterol absorption inhibitors (such as ezetimibe), bile acid sequestrants (such as cholestyramine and colestipol), fibric acid derivatives (such as fenofibrate and gemfibrozil) and high doses (3-6 g/day) of niacin. Although these drugs are effective to treat hypercholesterolemia, some patients suffer sever side-effects.

As mentioned above, there are a number of dietetic approaches to cure or ameliorate symptoms of cardiovascular diseases and obesity.

For improvement of the health status of an individual in general numerous food supplements are available. Usually they are based on the use of vitamins and other metabolically relevant substances. Some of these also claim to be beneficial for patients suffering from cardiovascular disease, obesity etc.

However, in the past, the common practice was to include numerous metabolically relevant ingredients in food supplements for improvement of nutrition and of the general well being. This led to compositions comprising dozens of substances without verification whether these substances in combination really provide beneficial effects for the patient or not. Even worse, sometimes the erratic combination of unlimited ingredients can cause serious adverse effects in the consumer. Therefore, it becomes more and more apparent that the ingredients in such compositions should preferably not be combined at random but have to be selected carefully and based on profound knowledge.

Thus, for the prevention and/or treatment of hypertriglyceridemia and hypercholesterolemia and related diseases such as arteriosclerosis and obesity there is still a need for drugs and compositions which are easy to administer and which are pharmaceutically acceptable and have no or little adverse effects. In particular drugs are needed which affect triglyceride and cholesterol levels in the blood of a patient.

Therefore, it is an object of the present invention to provide compositions effective in reducing blood triglyceride levels and/or cholesterol levels for the treatment and/or prophylaxis of hypertriglyceridemia, hypercholesterolemia and disease states induced thereby such as arteriosclerosis and obesity.

The object is solved by the subject-matter as defined in the claims.

The following figures are part of the present invention and are included to demonstrate certain aspects of the present invention. It has to be understood that the figures are not considered to be limiting the scope of the invention in any respect. However, the invention may be better understood by reference to these figures in combination with the detailed description of specific embodiments detailed further below.

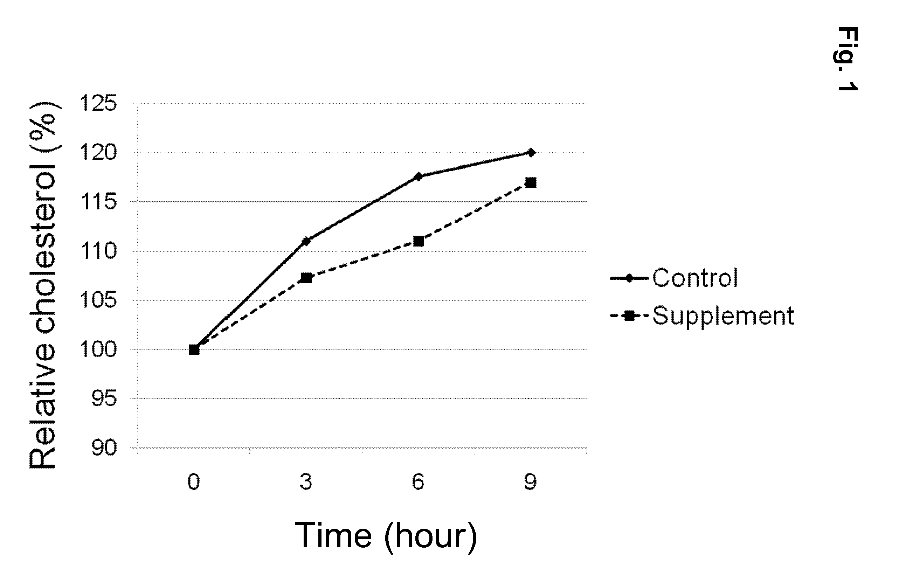

Figure 1: Effect of the composition according to the present invention on cholesterol levels of monkeys after a lipid challenge test. Blood samples were collected at 0, 3, 6, and 9 hours after loading of a lipid suspension. The plot shows relative change in blood cholesterol levels (%) over time (h) compared to the starting point 0. Supplement: Monkeys in this group received the supplement according to the present invention prior to the experiment. Control: Monkeys in this group did not receive the supplement according to the present invention prior to the experiment.

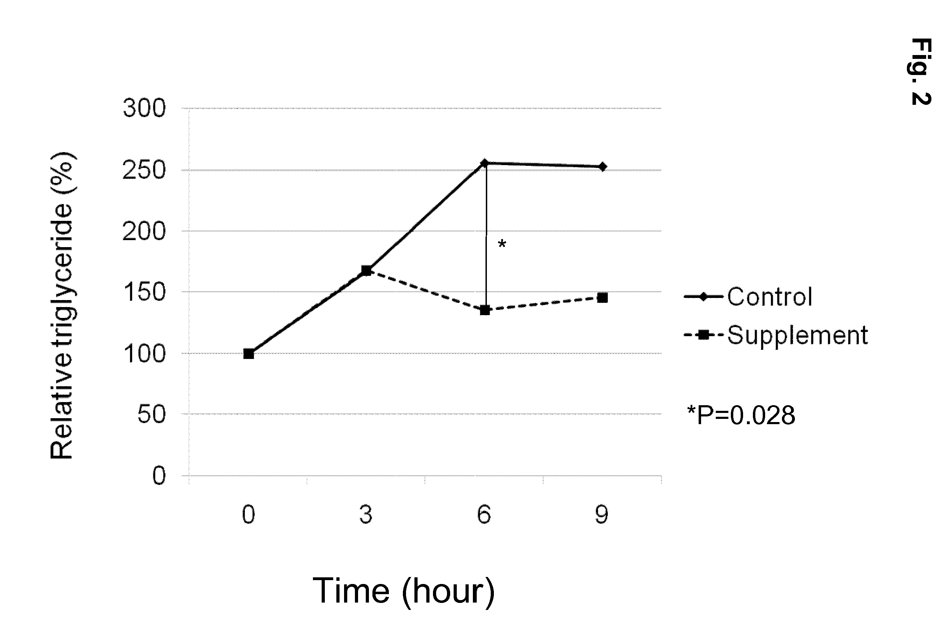

Figure 2: Effect of the composition according to the present invention on triglyceride levels of monkeys after a lipid challenge test. Blood samples were collected at 0, 3, 6, and 9 hours after loading of a lipid suspension. The plot shows relative change in blood triglyceride levels (%) over time (h) compared to the starting point 0. Supplement: Monkeys in this group received the supplement according to the present invention prior to the experiment. Control: Monkeys in this group did not receive the supplement according to the present invention prior to the experiment. *: P=0.031.

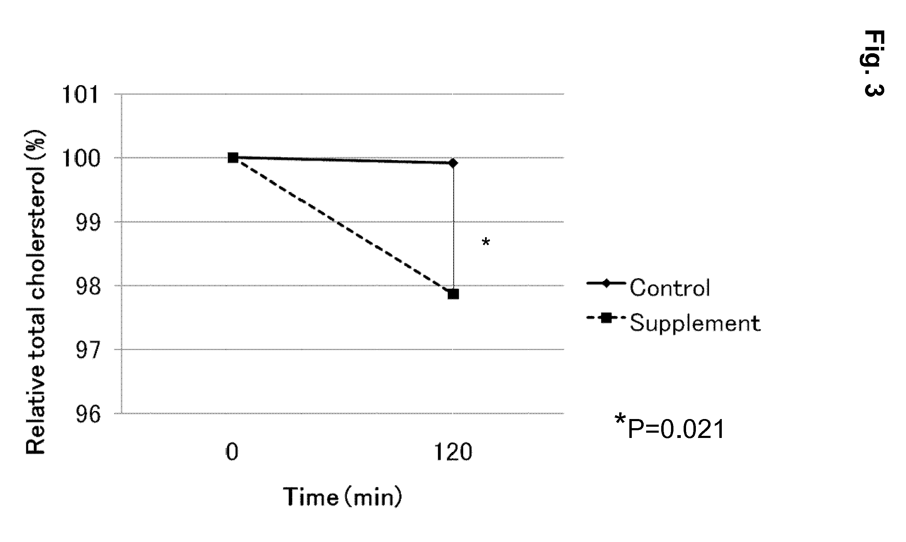

Figure 3: Effect of the composition according to the present invention on total cholesterol levels of humans after a snack bread test. Blood samples were collected at 0 and 120 minutes after consumption of snack breads. The plot shows relative change in blood cholesterol levels (%) over time (min) compared to the starting point 0. Supplement: Human volunteers in this group received the supplement according to the present invention prior to the experiment. Control: Human volunteers in this group did not receive the supplement according to the present invention prior to the experiment. *: P=0.021

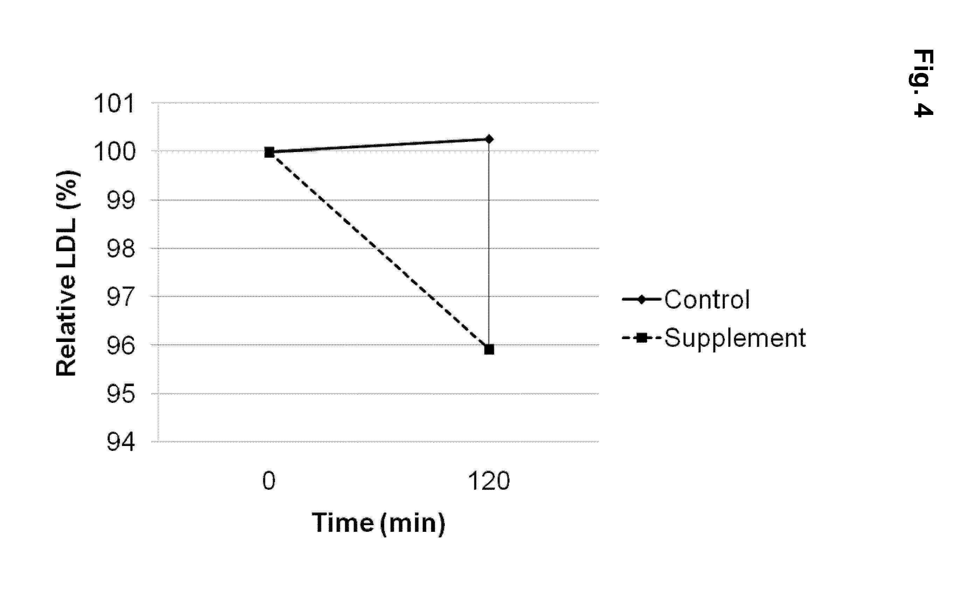

Figure 4: Effect of the composition according to the present invention on low density cholesterol (LDL) levels of humans after a snack bread test. Blood samples were collected at 0 and 120 minutes after consumption of snack breads. The plot shows relative change in blood LDL levels (%) over time (min) compared to the starting point 0. Supplement: Human volunteers in this group received the supplement according to the present invention prior to the experiment. Control: Human volunteers in this group did not receive the supplement according to the present invention prior to the experiment. *: P=0.01

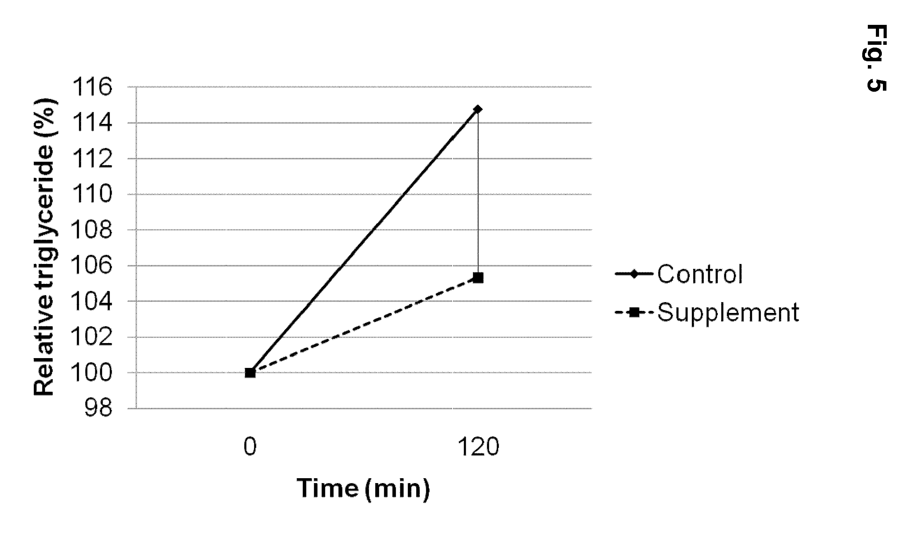

Figure 5: Effect of the composition according to the present invention on triglyceride levels of humans after a snack bread test. Blood samples were collected at 0 and 120 minutes after consumption of snack breads. The plot shows relative change in blood triglyceride levels (%) over time (min) compared to the starting point 0. Supplement: Human volunteers in this group received the supplement according to the present invention prior to the experiment. Control: Human volunteers in this group did not receive the supplement according to the present invention prior to the experiment. *: P=0.013
The term "hypertriglyceridemia", as used herein, refers to a condition in which elevated levels of blood triglycerides with regard to the normal average level of blood triglycerides in a respective reference subject of the same ethnic background, age and gender. With regard to humans the term in particular refers to blood triglyceride levels above about 150 mg/dl, in particular above about 180 mg/dl.
The term "hypercholesterolemia", as used herein, refers to a condition in which elevated levels of blood cholesterol with regard to the normal average level of blood cholesterol in a respective reference subject of the same ethnic background, age and gender. With regard to humans the term in particular refers to blood cholesterol levels above about 200, in particular above about 240 mg/dl (USA, National Cholesterol Education Program NCEP, 1987).

As used herein, the terms "hypertriglyceridemia-related disease state" and "hypercholesterolemia-related disease state" refer to all those disease conditions which can be caused by and have as a symptom elevated blood triglyceride levels and/or blood cholesterol levels, respectively. The diesease states have in common that the elevated triglyceride levels and/or blood cholesterol levels are detrimental for the subject. Hypertriglyceridemia-related disease states can be for instance atherosclerosis, pancreatitis, heart diseases, stroke, insulin resistance, metabolic syndrome, high blood pressure and obesity. Hypercholesterolemia-related disease states can be for instance atherosclerosis, cardiovascular diseases, myocardial infarction, stroke, angina pectoris, ischemic colitis, transient ischemic attacks, and/or peripheral artery disease. As used in this application, neither hypertriglyceridemia-related disease state nor hypercholesterolemia-related disease state refers to diabetes.

Dosage form, as used herein, refers to an amount of medication to be taken at one time, optionally in regular intervals.

The term "supplement", "dietary supplement" or "food supplement" as used herein, refers to a composition which is consumed in addition to meals or drinks. Consumption of the supplement can occur before, simultaneously or after uptake of the meal or drinks. If consumption occurs simultaneously to the meal or drink, then the composition can be an ingredient, i.e. additive of the respective meal or drink itself, or can be consumed from a source different than the meal or drink. Ingredient, as used herein, is a substance or composition, which forms part of a composition. In particular this term applies, if the composition for use in the present invention is not a medicament, i.e. pharmaceutical composition.

The term "prodrug" as used herein refers to a substance which is converted into at least one of the active ingredients of the composition of the present invention as a consequence of metabolisation after uptake by a human or animal. The term is not intended to refer to compounds which yield after metabolisation only to a negligible extent one of the active substances of the composition of the present invention. For example, proteins and peptides are digested during metabolisation into individual amino acid. Thus, while a protein or peptide rich in glutamic acid, glutamine and/or cysteine can function as prodrug according to the invention, proteins or peptides with only about average or slightly elevated frequency of glutamic acid, glutamine and/or cysteine are not considered to be a prodrug for said substances. In particular the term "prodrug" refers to a compound which is metabolised into the active substance within 5, preferably 4, even more preferred 3, even more preferred 2, and most preferred within 1 metabolic reaction(s).

The term "treatment", as used herein, comprises curing a respective disease as well as ameliorating symptoms associated with the respective disease.

According to the present invention the object is attained by using a composition comprising the following (active) substances:
- Vitamin C and/or a prodrug thereof and/or a salt thereof,
- glutamic acid and/or glutamine, and/or prodrugs of these and/or salts of these,
- cysteine, and/or a prodrug thereof and/or a salt thereof,
- riboflavin, and/or a prodrug thereof and/or a salt thereof,
- succinic acid, and/or a prodrug thereof and/or a salt thereof,
- fumaric acid, and/or a prodrug thereof and/or a salt thereof,
- coenzyme Q10, and/or a prodrug thereof and/or a salt thereof, and
- niacin, and/or a prodrug thereof and/or a salt thereof,
for the manufacture of a food supplement or medicament for the treatment and/or prophylaxis of hypertriglyceridemia and hypercholesterolemia and related disease states as well as of erectile dysfunction.

Vitamin C, also called ascorbic acid is used by the body for many purposes. By far its main purpose is to function as reducing agent. For the compositions of the present invention Vitamin C (ascorbic acid) as well as its salt ascorbate can be applied.

Glutamic acid is a non-essential amino acid. It plays an important role in human metabolism and can function as neurotransmitter. Because of the latter it is often used in food supplements. For the compositions of the present invention glutamic acid as well as its salt glutamate can be applied.

Glutamine is a non-essential amino acid which plays, besides being an important component of proteins, an important role in the nitrogen metabolism. It is used as a supplement in weightlifting, bodybuilding, endurance and other sports, as well as by those who suffer from muscular cramps or pain-particularly by elderly people. For the compositions of the present invention glutamine as well as a salt of glutamine can be applied.

Cysteine is a naturally occurring amino acid which has a thiol group and is found in most proteins. When it is exposed to air, cysteine oxidizes to form cystine, which is a dimer of two cysteine molecules joined by a weak disulfide bond. Because it is a sulphur-based amino acid, cysteine itself can act as an antioxidant in the body. Cysteine is an important source of sulphur in human metabolism, and although it is classified as a non-essential amino acid, cysteine may be essential for infants, the elderly, and individuals with metabolic disease or who suffer from malabsorption syndromes. Cysteine may at some point be recognized as an essential or conditionally essential amino acid. For the compositions of the present invention cysteine, salts thereof as well as cystine can be applied. The latter functions as exemplary prodrug as defined by the present invention.

Riboflavin (Vitamin B2) is an essential compound for higher animals including humans. Riboflavin is commercially used as a vitamin preparation for use in vitamin deficiency and as a food supplement. In addition, it is also employed as a food dye, for example in mayonnaise, ice cream etc. Riboflavin can be prepared either chemically or microbiologically and can be obtained from several manufacturers. Biologically active riboflavin is flavin mononucleotide (FMN) or flavin adenine dinucleotide (FAD). These active forms and their reduced forms, FMNH2 and FADH2 can also be applied for use in the present invention.

Succinic acid is also termed butanedioic acid, amber acid or E 363 and is used as food supplement, for example as substitute for cooking salt in dietary meals or as flavour enhancer. In the citric cycle the salt of the succinic acid, succinate, is involved in regeneration of the acceptor oxalate. For the compositions of the present invention succinic acid as well as succinate, and also its anhydrated form succinic anhydride can be applied.

Fumaric acid, also termed 2-butenedioic acid, allomaleic acid, boletic acid or lichenic acid, is used as flavouring and thus is a common component of food additives and dietary supplements. For the compositions of the present invention fumaric acid as well as its salt fumarate can be applied.

Coenzyme Q10, also known as CoQ10 as well as ubiquinone-10 or ubiquinone 50, is the most common CoQ in human mitochondria. There it is involved in the electron transport chain. It is used as supplement because of its antioxidant function.

Niacin is also known as nicotinic acid (nicotinate) or Vitamin B3. The term also comprises the amide form, nicotinamide or niacinamide. Its biologically acitive forms nicotinamide adenine dinucleotide (NAD) and nicotinamide adenine dinucleotide phosphate (NADP) as well as their reduced forms NADH and NADPH play essential roles in the metabolism of any living being. Nicotinamide mononucleotide (NMN), desamido-NAD and deamino-NAD (also known as nicotinamide hypoxanthine dinucleotide) are metabolic intermediates containing the niacin molecule. All of these forms can be used for the compositions of the present invention. NAD, NADH, NADP, NADPH, NMN, deamido-NAD, and/or deamino-NAD containing niacin molecules can also be applied.

Proteins and peptides, i.e. polymers of amino acids are easily digested in the small intestine. Therefore, proteins and peptides consisting to a significant extent of cysteine, glutamic acid and/or glutamine and combinations thereof can also be applied. For example, proteins and peptides having a amino acid frequency of 10, 15, 20, 25, 30, 35, 40, 50 or more percent of cysteine, glutamine and/or glutamic acid can function as prodrug according to the present invention and thus can be used - alternatively or in addition to glutamic acid, glutamine and/or cysteine - for the manufacture of a food supplement or medicament for the treatment and/or prophylaxis of hypertriglyceridemia and hypercholesterolemia and related disease states as well as erectile dysfunction. Such proteins and peptides are further examples for prodrugs as defined by the present invention.

In a further embodiment the present invention relates to the use of one or more of the substances selected from vitamin C, glutamic acid, glutamine, cysteine, riboflavin, succinic acid, fumaric acid, coenzyme Q10, niacin, and/or prodrugs of these substances and/or salts of these substances for the manufacture of a medicament comprising vitamin C, glutamic acid and/or glutamine, cysteine, riboflavin, succinic acid, fumaric acid, coenzyme Q10 and niacin, and/or prodrugs of these substances and/or salts of these substances for the treatment and/or prevention of hypertriglyceridemia and hypercholesterolemia and related disease states as well as erectile dysfunction.

In a further aspect the present invention relates to the use of a food supplement comprising the composition according to the present invention for prophylaxis and treatment of hypertriglyceridemia and hypercholesterolemia and related disease states as well as of erectile dysfunction.

The composition for use can comprise, if desired, further substances, such as saccharides, vitamins, amino acids or other metabolically important substances. However, in a particular embodiment the food supplement comprises the composition according to the present invention but no further vitamins, no further amino acids, no further organic acids, no further nucleotides, no polysaccharides, no oligosaccharides, no disaccharides and/or no monosaccharides, in particular no glucose, no triglycerides. In a further embodiment of the invention the composition consists only of vitamin C, glutamic acid and/or glutamine, cysteine, riboflavin, succinic acid, fumaric acid, coenzyme Q10, niacin and/or prodrugs of these substances and/or salts of these substances plus optionally suitable carrier substances. The use of the composition of the present invention as food supplement allows an easy way to profit from the advantageous effects of the composition of the present invention on blood triglyceride and cholesterol levels. The use of this food composition is not restricted to human use only but can also be used for animal food, in particular pet food due to the biological importance of the substances of the composition for use in the present invention not only for man but for animals as well.

If the composition for use in the present invention is to be administered as a food supplement or dietary supplementation, it is preferably an ingredient of a kind of drink, that allows the composition according to the present invention to be consumed before, simultaneously or after consumption of food or drinks, in particular after consumption of food or drinks with triglyceride and/or cholesterol content. Such drink unit can comprise a dosage of the composition according to the present invention sufficient to affect blood triglyceride levels and/or blood cholesterol levels and to reduce them, in the optimal case, quickly to normal levels.

Alternatively, the food supplement for use in the present invention is consumed as tablets or capsules. Preferably, each tablet and/or capsule is so shaped and dimensioned that it allows said tablet and/or capsule to be easily swallowed. Such tablet and/or capsule can comprise a dosage sufficient to moderate blood triglyceride levels and/or blood cholesterol levels after a meal. But preferably said tablets and/or capsules are in such a form that one dosage includes a plurality of tablets and/or capsules. The tablets and/or capsules may be accommodated within a dosage receptacle which includes a number of those tablets and/or capsules. The food supplement or dietary supplementation may be separated into separate subunits, i.e. not all ingredients and/or substances of the composition of the present invention have to be within the same capsule or tablet. For example, it is possible to provide one unit, for example a capsule including the vitamin C fraction, cysteine, riboflavin, succinic acid, fumaric acid and coenzyme Q10, whilst the rest of the substances of the composition of the present invention is kept in separate units, capsules, tablets or the like. However, preferably all the different subunits are consumed simultaneously or in close time intervals. The tablets or capsules can also comprise suitable carrier substances examples of which will be known to the skilled artisan.

In another embodiment the food supplement according to the present invention is of a cube type form.

The food supplement can also be in the form of a powder, in particular a cryopowder, which can be added to the meal and/or drink directly or which is dissolved for example in water prior to use.

The food supplement can also be present in form of a liquid, in particular as a syrup-type liquid, which can be added to the meal or drink, and/or which can be consumed on its own. In a particular embodiment the food supplement is dissolved in water.

If the food supplement is already an ingredient of the meal or drink, then the dosage of the composition according to the present invention is preferably calculated to moderate blood triglyceride levels and/or blood cholesterol levels resulting from uptake of the respective meal and/or drink.

It is possible to add further substances to the food supplement such as sweetening, flavouring, preservatives, stabilizers, colourings and pigments. Exemplary additives are fruit juice extracts, curcuma, tannin, a powder of Panax notoginseng, and Vinca rosea in suitable amounts. Oolong tea, aloe vera and spiral water algae might also be added.

An exemplary dosage of the composition of the present invention comprises niacin (vitamin B3), vitamin C, glutamine and/or glutamic acid, cysteine, riboflavin (vitamin B2), succinic acid, fumaric acid, and coenzyme Q10 and/or prodrugs of these substances and/or salts thereof in physiologically relevant amounts. The food supplement can be prepared in such manner that it is compatible with a diet.

The food supplement or medicament for the treatment and/or prophylaxis of hypertriglyceridemia and hypercholesterolemia and related disease states as well as erectile dysfunction is of use for human as well as for veterinary medicine. In human medicine the food supplement or medicament can be administered to any kind of patient in need thereof, irregardless of age and gender. In veterinary medicine for example cats, dogs, birds, cattle, horses, rodents and so forth can be treated, as these animals are also sometimes treated when suffering from hypertriglyceridemia and hypercholesterolemia and related disease states. However, in principle the composition according to the present invention can be administered to any kind of animal as demonstrated by the experiments with Cynomolgus Monkeys exemplified further below.

In a further embodiment the composition of the present invention is used for the manufacture of a pharmaceutical composition for the reduction of elevated blood triglyceride levels and/or cholesterol levels in the blood of a subject, preferably for the prophylaxis and/or treatment of hypertriglyceridemia and hypercholesterolemia related disease conditions such as atherosclerosis, pancreatitis, cardiovascular diseases, stroke, insulin resistance, metabolic syndrome, high blood pressure, obesity, myocardial infarction, stroke, angina pectoris, ischemic colitis, transient ischemic attacks, and/or peripheral artery disease and for reducing the risk for onset or progression of the aforementioned diseases by moderating the triglyceride and cholesterol metabolism within the human body.

The fact, that elevated blood triglyceride and/or cholesterol levels, a common feature for example of the above mentioned disease states, are much more rapidly cleared from the blood when using the food supplement or medicament of the present invention makes the composition of the present invention ideal for the treatment of all those disease states associated with hypertriglyceridemia and/or hypercholesterolemia.

The pharmaceutical compositions for use in the invention can be employed in the form of pharmaceutical preparations. In such an embodiment the pharmaceutical composition comprises the composition as mentioned above and additionally a pharmaceutically acceptable carrier. Such preparations are made in a manner well known in the pharmaceutical art. A preferred preparation utilizes a vehicle of physiological saline solution, but it is contemplated that other pharmaceutically acceptable carriers such as physiological concentrations of other non-toxic salts, sterile water or the like may also be used. It may also be desirable that a suitable buffer be present in the composition. Such solutions can, if desired, be lyophilized and stored in a sterile ampoule ready for reconstitution by the addition of sterile water for ready injection.

In one embodiment such pharmaceutical compositions are preferably formulated with suitable carriers. Some examples of suitable carriers, excipients, and diluents include lactose, sorbitol, mannitol, gum acacia, calcium phosphate, alginates, calcium silicate, microcrystalline cellulose, polyvinylpyrrolidone, cellulose, gelatine, syrup, methyl cellulose, methyl-and propylhydroxybenzoates, talc, magnesium, stearate, water, mineral oil, and the like. The formulations can additionally include lubricating agents, wetting agents, emulsifying and suspending agents, preserving agents, sweetening agents or flavouring agents. The compositions may be formulated so as to provide rapid, sustained, or delayed release of the active ingredients after administration to the patient by employing procedures well known in the art. The formulations can also contain substances that diminish proteolytic degradation and promote absorption such as, for example, surface active agents.

The carrier can also contain other pharmaceutically-acceptable excipients for modifying or maintaining the pH, osmolarity, viscosity, clarity, color, sterility, stability, rate of dissolution, or odor of the formulation. Similarly, the carrier may contain still other pharmaceutically-acceptable excipients for modifying or maintaining release or absorption or penetration across the blood-brain barrier. Excipients can be also those substances usually and customarily employed to formulate dosages for parenteral administration in either unit dosage or multi-dose form or for continuous or periodic infusion.

Preferably, the pharmaceutical composition for use in the present invention is administered to the subject at a suitable dose. Administration of the suitable compositions may be effected by different ways, e.g. oral, intravenous, intraperitoneal, subcutaneous, intramuscular, topical or intradermal.

If the pharmaceutical composition is to be orally administered it is preferably of similar form as discussed above for the food supplement.

The pharmaceutical composition for use in the present invention can be administered when the blood triglyceride levels and/or blood cholesterol levels are already elevated, but can also be administered in advance, if the blood triglyceride levels and/or blood cholesterol levels are expected to rise in the near future or if any potential rise of the blood triglyceride levels and/or blood cholesterol levels would be detrimental for the health and/or status of the patient. In the latter case (detrimental effect) the pharmaceutical composition for use in the present invention is in particular administered to anticipate and prevent peaks of blood triglyceride levels and/or blood cholesterol levels.

In a particular embodiment the pharmaceutical composition comprising the composition as mentioned above allows for the combined administration or application with further (a) pharmaceutical composition(s) and/or method(s) of treatment. For example, the further pharmaceutical composition(s) or method of treatment(s) can be selected - taking into account the particular disease to be treated - from the following group: fat absorption inhibitors blocking for example pancreatic triglyceride lipase in the intestine, (e.g. Orlistat); thermogenic agents, such as thyroid hormones and/or 3-adrenergic agonists; anorectics, such as serotonin agonists, sympathomimetic agents and/or leptin; HMG-CoA reductase inhibitors; cholesterol absorption inhibitors (e.g. ezetimibe); bile acid sequestrants (e.g. cholestyramine and colestipol) and fibric acid derivatives (e.g. fenofibrate and gemfibrozil).

In a particular embodiment of the invention the pharmaceutical composition for use in the present invention is also compatible with physical exercise, a diet and/or dietary therapy approaches to reduce or prevent hypertriglyceridemia and/or hypercholesterolemia. Such diets or dietary therapies relate e.g. to the reduction of caloric intake, fat intake and/or cholesterol intake.

In a particular embodiment the composition, the food supplement or the pharmaceutical composition for use in the present invention preferably comprises vitamin C in the range from about 0.01 g to about 50 g, preferably about 0.1 g to about 10 g, preferably about 1.0 g.

In a preferred embodiment the composition, the food supplement or the pharmaceutical composition for use in the present invention preferably comprises a glutamic acid or glutamine fraction in the range from about 0.01 g to about 50 g, preferably about 0.1 g to about 10 g, preferably about 1.5 g.

In a preferred embodiment the composition, the food supplement or the pharmaceutical composition for use in the present invention preferably comprises a cysteine fraction in the range from about 0.01 g to about 50 g, preferably about 0.1 g to about 10 g, preferably about 500 mg.

In a preferred embodiment the composition, the food supplement or the pharmaceutical composition for use in the present invention preferably comprises riboflavin in the range from about 0.001 g to about 50 g, preferably about 0.01 g to about 1 g, preferably about 40 mg.

In a preferred embodiment the composition, the food supplement or the pharmaceutical composition for use in the present invention preferably comprises succinic acid in the range from about 0.001 g to about 50 g, preferably about 0.01 g to about 1 g, preferably about 100 mg.

In a preferred embodiment the composition, the food supplement or the pharmaceutical composition for use in the present invention preferably comprises fumaric acid in the range from about 0.001 g to about 50 g, preferably about 0.01 g to about 1 g, preferably about 100 mg.

In a preferred embodiment the composition, the food supplement or the pharmaceutical composition for use in the present invention preferably comprises a coenzyme Q10 fraction in the range from about 0.001 g to about 50 g, preferably about 0.01 g to about 1 g, preferably about 100 mg.

In a preferred embodiment the composition, the food supplement or the pharmaceutical composition for use in the present invention preferably comprises niacin in the range from about 1 mg to about 30 mg, preferably about 5, 10, 15 or 20 mg.

Preferably the composition, the food supplement or the pharmaceutical composition for use in the present invention is formulated to be consumed by a subject in need thereof on a daily basis.

In a further aspect the present invention relates to an aaphrodisiac comprising a composition comprising Vitamin C, glutamic acid and/or glutamine, cysteine, riboflavin, succinic acid, fumaric acid, coenzyme Q10, and niacin, and/or prodrugs of these substances and/or salts of these substances. The inventors of the present application discovered that, in addition to positive effects on blood triglyceride and blood cholesterol levels, the composition for use in the present invention has positive impact on virility. Consequently the composition for use in the present invention can be used also as aphrodisiac.

Other embodiments within the scope of the claims herein will be apparent to one skilled in the art from consideration of the specification or practice of the invention as disclosed herein. It is intended that the specification, together with the examples, be considered exemplary only, with the scope and spirit of the invention being indicated by the claims which follow the examples.

The invention will now be described in more detail by way of the following non-limiting examples.

### Examples:

### Example 1: Supplement

Ingredients of Supplement except were purchased from Sigma Aldrich Japan (Tokyo, Japan). AQUAQ10P40 (Nissin Pharma, Tokyo Japan), which contains 40% by volume of coenzyme Q10, was used as coenzyme Q10. One gram of Vitamin C, 1.5 gram of glutamic acid, 500 mg of cysteine, 40 mg of riboflavin, 100 mg of succinic acid, 100 mg of fumaric acid, and 10 mg of niacin, and 250 mg of AQUAQ10P40 (100 mg of coenzyme Q10) were mixed. Total weight for one portion is 3,500 mg.

### Example 2: Effect of the composition of the present invention on blood cholesterol and triglyceride levels of Cynomolgus Monkey after oral lipid challenge experiments:

Cynomolgus monkeys underwent oral lipid challenge experiments in two groups, either with or without prior administration of the supplement as mentioned under Example 1. 3 animals per group were used. For each animal which received the supplement, one portion (3,500 mg) of supplement was dissolved in 70 ml of distilled water, and 100 mg/Kg of supplement solution were administrated via gastric intubation to each of the respective animals 10 minutes before loading of 150 mg/Kg of a lipid suspension also via gastric intubation (3.5 g of Taurocholic acid sodium salt and 2.0 g of Cholesterol suspended in 20 ml of chicken oil). Blood samples were collected at 0, 3, 6, and 9 hours after loading the lipid suspension, and blood cholesterol levels as well as triglyceride levels were determined. Statistical analysis of the results was conducted by t2 test, and p<0.05 was defined as the level of significance.

The challenge with the lipid suspension led in both animal groups to an increase in blood cholesterol levels as well as in blood triglyceride levels. However, the increase in blood cholesterol was less prominent and constantly lower in animals having received the supplement prior to loading of said lipid suspension than in animals which had not received said supplement. In addition, animals which had received the supplement showed after 6 hours and later a much lower increase in blood triglyceride levels than animals of the control group which had not received the supplement.

In summary, Cynomolgus monkeys which received the supplement according to the present invention showed a reduction in blood triglyceride and blood cholesterol levels in comparison to control animals.

### Example 3: Effect of the composition of the present invention on blood cholesterol and triglyceride levels of humans after a snack bread challenge test.

Fourteen healthy volunteers, i.e. 11 males and 3 females, age 29-60 (average age 47), not suffering form hypertriglyceridemia or hypercholesterolemia, had two consecutive snack breads tests, once with and once without prior administration of the supplement as mentioned under Example 1. The two experiments were conducted on different days. In the experiment with prior administration of the supplement, the supplement was taken with 150 ml of water 20 minutes prior to eating snack breads. For the snack bread test, two breads with adzuki paste were used. Total nutrition facts of two breads are in calories: 800 Kcal, protein: 20 gram, fat: 8 gram, and carbohydrate: 130 gram, respectively. Two snack breads were eaten by each volunteer within 20 minutes, and blood samples were taken at 0, and 120 minutes after volunteers started to eat the breads. The blood samples were centrifuged immediately after sampling, and levels of total cholesterol, low density cholesterol (LDL), high density cholesterol (HDL) and triglycerides, as well as liver and kidney functions were determined. Statistical analysis of the results was conducted by t2 test, and p<0.05 was defined as the level of significance.

In the experiment where the volunteers had received the supplement, blood total cholesterol levels were 120 minutes after consumption of the snack breads significantly (P=0.021) lower than in the experiment without the supplement. This enhanced decrease in blood cholesterol is advantageous because cholesterol is a direct risk factor for, e.g., atherosclerosis and cardiovascular diseases.

In more detail, consumption of the supplement prior to consumption of the snack breads yielded a pronounced decrease in LDL cholesterol. The results is statistically significant (P=0.01).

In addition to a decrease in blood cholesterol levels, uptake of the supplement leads also to a reduced increase in blood triglyceride levels, keeping the blood triglyceride levels always lower than in the experiment where no supplement had been consumed. The result was statistically significant (P=0.013).

Finally, in none of the volunteers a significant difference in liver and kidney function between 0 and 120 minute was observed, irregardless whether the supplement had been received or not. This indicates that there are no problematic side effects to be expected from consumption of the supplement of the present invention.

In summary, consumption of the supplement of the present invention had consistently positive effects on blood triglyceride and blood cholesterol levels and consequently is helpful in moderating detrimental elevations in these parameters.

### Example 4: Aphrodisiac effects of the supplement of the present invention

Ten healthy male volunteers, i.e. age 32-60 (average age 45) took supplement and aphrodisiac effects was monitored by themselves. In the experiment with prior administration of the supplement, the supplement was taken with 150 ml of water and effect on erection was observed. Eight volunteers (80%) reported unexpected erection which started 10-120 minutes after consumption of the supplement. The experiments were repeated 3 times, and volunteers who report positive effect are proved same effects in all experiments.

## Claims

1. Use of a composition comprising Vitamin C, glutamic acid and/or glutamine, cysteine, riboflavin, succinic acid, fumaric acid, coenzyme Q10, and niacin, and/or prodrugs of these substances and/or salts of these substances for the manufacture of a medicament for the prophylaxis and/or treatment of hypertriglyceridemia, hypercholesterolemia, hypertriglyceridemia related disease states, hypercholesterolemia related disease states, as well as of erectile dysfunction.

2. The use according to claim 1, wherein the hypertriglyceridemia- and hypercholesterolemia-related disease states are selected from atherosclerosis, obesity, metabolic syndrome, high blood pressure, pancreatitis, cardiovascular diseases, stroke, mycardial infarction, angina pectoris, ischemic colitis, transient ischemic attacks, and/or peripheral artery disease.

3. Use of a composition comprising Vitamin C, glutamic acid and/or glutamine, cysteine, riboflavin, succinic acid, fumaric acid, coenzyme Q10, and niacin, and/or prodrugs of these substances and/or salts of these substances for the manufacture of a food supplement for the prophylaxis and/or treatment of hypertriglyceridemia, hypercholesterolemia, hypertriglyceridemia related disease states, hypercholesterolemia related disease states, as well as of erectile dysfunction.

4. The use according to claim 3, wherein the hypertriglyceridemia- and hypercholesterolemia-related disease states are selected from atherosclerosis, obesity, metabolic syndrome, high blood pressure, pancreatitis, cardiovascular diseases, stroke, mycardial infarction, angina pectoris, ischemic colitis, transient ischemic attacks, and/or peripheral artery disease.

5. The use according to anyone of claims 1 to 4, wherein the composition is present in dosage form, as powder, as liquid, in particular as drink unit or as syrup.

6. The use according to claim 5, wherein the dosage form is in particular in form of tablets or in cube type form or wherein a dose comprises a tablet or capsule or a plurality of tablets or capsules.

7. The use according to claim 5, wherein the tablets or capsules are present in a dosage receptacle.

8. Use according to anyone of the previous claims, wherein the manufactured medicament allows for the combined administration with one or more of the following medicaments: fat absorption inhibitors, in particular. Orlistat; thermogenic agents, in particular thyroid hormones and/or 3-adrenergic agonists; anorectics, in particular serotonin agonists, sympathomimetic agents and/or leptin; HMG-CoA reductase inhibitors; cholesterol absorption inhibitors, in particular ezetimibe; bile acid sequestrants in particular cholestyramine and/or colestipol and/or fibric acid derivatives, in particular fenofibrate and/or gemfibrozil.

9. Aphrodisiac comprising a composition comprising Vitamin C, glutamic acid and/or glutamine, cysteine, riboflavin, succinic acid, fumaric acid, coenzyme Q10, and niacin, and/or prodrugs of these substances and/or salts of these substances.
